# EUROPEAN PATENT APPLICATION

(11) **EP 0 955 288 A2**
(43) Date of publication of application: **10.11.1999**
(21) Application number: 99108329.6
(22) Date of filing: 28.04.1999
(51) Int. Cl.: C07C 211/63, A61K 7/48, C07C 65/10, C07C 63/08, C10M 105/28, C10M 105/58, A01N 33/12

(54) **Dialkylquat and trialkylquat benzoate and salicylate salts**

(30) Priority: 05.05.1998 US 72818
(71) Applicant: WITCO CORPORATION, Greenwich, Connecticut 06831-2559 (US)
(72) Inventor: Zhu, Shawn, Dublin, OH 43017 (US)
(74) Representative: Graalfs, Edo, Dipl.-Ing. Patentanwälte Hauck, Graalfs, Wehnert, Döring, Siemons

(57) **Abstract**

Quaternary complex aromatic acid salts of the formula: 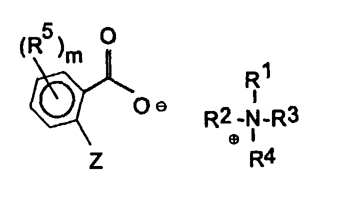 where R¹ is an alkyl group having 1 to 4 carbon atoms, which may optionally contain a hydroxyl group, R² and R³ are independently alkyl or alkenyl groups, which may be optionally interrupted with ester, ether oxygen and/or amido linkages, the R² and R³ groups having a total of 8 to about 30 carbon atoms, R⁴ is a group as defined for R¹ or for R², Z is H or OH, R⁵ is alkyl, alkaryl, aralkyl, halo, alkoxy, hydroxy and m is 0-4, are stable in aqueous dispersion over a wide range of pH. The compounds are effective as emollients, lubricants and hydrophobes, and can be used in skin care and lubricant compositions, as carriers for biological actives and for other purposes.

## Description

### Background of the Invention

### Definitions

For purposes of this invention the following definitions are used:
Monoalkylquat salt. A quarternized ammonium compound having attached to the quarternized nitrogen atom thereof, a single long chain aliphatic hydrocarbon group with at least 8 carbon atoms, which may be optionally interrupted with amido, ether oxygen and/or ester functional groups, and three lower (C₁₋₆) alkyl groups, which may optionally contain a hydroxy group.
Dialkylquat salt. A quarternized ammonium compound, having attached to the quarternized nitrogen atom thereof, two long chain aliphatic hydrocarbon groups with at least 8 carbon atoms, which may be optionally interrupted with amido, ether oxygen and/or ester functional groups, and two lower alkyl groups, which may optionally contain a hydroxy group.
Trialkylquat salt. A quarternized ammonium compound having attached to the quarternized nitrogen atom thereof three long chain aliphatic hydrocarbon groups, which may be optionally interrupted with amido, ether oxygen and/or ester functional groups, and a single lower (C₁₋₄) alkyl group, which may optionally contain a hydroxy group.

### Description of Prior Art

US 3,940,339 describes a lithium borate complex grease, prepared from 12-hydroxy stearic acid, lithium hydroxide, boric acid, methyl salicylate, solvent oil, dicoco dimethyl ammonium nitrate, amino imidazoline.

US 4,162,984 describes and claims textile treatment formulation in the form of aqueous dispersion which includes a water insoluble cationic fabric softener which may be a dialkylquat salt; a water insoluble nonionic fabric softener; and 0.1-10% of an aromatic acid as a thickener.

US 4,459,185 describes an alloy plating bath which includes a tin or lead salt, an alkanolsulfonic acid, a surfactant, which may be a quarternary ammonium salt, and a leveling agent which may be salicylic acid or an ester thereof.

US 4,615,825 describes aqueous compositions having reduced resistance to flow in a conduit. The compositions contain a monoalkylquat salicylate and additional sodium salicylate to form a viscoelastic cationic surfactant.

EP 0637625 describes fabric softener compositions which include a mixture of benzoic and salicylic acid, a quarternized ester ammonium salt surfactant which may be a mixture of monoalkylquat and dialkylquat salts, as well as other conventional fabric softener ingredients such as nonionic surfactants. A pH range of 2 to 5, more preferably 2 to about 4, is needed for stability.

WO 95/24179 and WO 95/03781 describe compositions for skin care which leave an acid active component such as salicylic acid on skin. According to these references the pH should be 3.5 or less. For salicylic acid formulations the pH should be 2.97 or less in order to maintain at least 50 % of the salicylic acid in non-ionized form. A cationic surfactant and a non-ionic surfactant is included in these formulations. The cationic surfactant used as an emulsifier may be a monoalkylquat salt or a dialkylquat salt.

WO 94/02176 describes formulations of pharmaceutical active, a cationic polymer, a non-ionic surfactant and an alkoxylated ether. For compositions employing salicylic acid as a pharmaceutical active, an acidic range is taught.

US 5,500,138 describes fabric softener compositions incorporating cationic softening compounds and optionally a pH modifier such as salicylic acid to adjust pH to 2-5, pref 2-4. A perfume may be incorporated with a salicylate ester used as a component of the perfume.

In US 4,795,860 and US 5,356,542 mono quat salts of salicylic acid are described as drag reducing agents for reducing pump energy requirements to move liquids in pipelines.

US 5,551,516 describes hydraulic fracturing composition for use in wellbore applications. A monoalkylquat salt is used as thickener in a formulation which also includes a sodium salicylate.

US 5,643,534 describes a method for inhibiting corrosion of metals in contact with an aq alkanolamine solution which uses a composition containing salicylic acid, a reaction product of maleated tall oil fatty acid polyhydroxy alkane and ammonium hydroxide, tallow diamine quaternary dichloride, an alkanol or alkanolamine water and an antifoaming agent.

US 5,019,567, US 5,001,156 and US 5,137,923 describe quat salts of 5-carboxylalkyl substituted salicylic acid for treatment of dermatoses such as acne or dandruff.

While monoalkylquat salicylates are known from some of the above described references, and in other of the above described references benzoic acid, salicylic acid or a salicylate salt has been incorporated or suggested to be incorporated into some prior formulations with a dialkylquat or trialkylquat salt, it has not previously been reported or suggested to prepare a dialkylquat or trialkylquat benzoate or salicylate compound *per se* and no reference has suggested that dialkylquat benzoate or salicylate or trialkylquat benzoate or salicylate compounds would have any uniquely beneficial properties over a monoalkylquat salicylate compound.

### Summary of the Invention

The present invention is directed to dialkylquat and trialkylquat compounds which are salts of benzoate or substituted benzoate anions, especially salicylate anions, and to compositions thereof and novel uses therefor.

While it is well known that typical mono, di and trialkylquat salts share many common physical properties, it has now been found that certain dialkylquat and trialkylquat benzoate and salicylate compounds have unique physical properties, compared to monoalkylquat counterparts: In particular, unlike monoalkylquat salicylates which thicken aqueous solutions, forming clear viscoelastic solutions as disclosed in US 4,615,825, US 4,795,860, US 5,356,542 and US 5,551,516, multialkylquat salicylates are essentially insoluble in water. Further they exhibit unique, unexpectedly high water repellancy and low friction properties which are not characteristic of monoalkylquat salicylates. These properties provide unusually beneficial characteristics to compositions employing the multialkylquat compounds, including skin feel, high water repellancy and lubricant characteristics.

The compounds of the invention comprise quaternary aromatic acid salts of the formula: 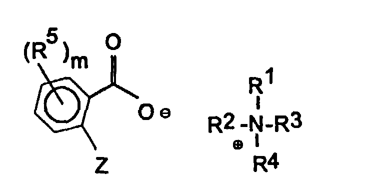 where R¹ is an alkyl group having 1 to 4 carbon atoms, which may optionally contain a hydroxyl group, R² and R³ are independently alkyl or alkenyl groups, which may be optionally interrupted with ester, ether and/or amido linkages, the R² and R³ groups each having a total of 8 to about 30 carbon atoms, R⁴ is a group as defined for R¹ or for R², Z is H or OH, R⁵ is alkyl, alkaryl, aralkyl, halo, alkoxy, hydroxy and m is 0-4. These compounds are tight ionic complexes which, after formation, are stable in aqueous environments over a wide range of pH. The compounds are very effective as emollients, lubricants and hydrophobes.

The invention also pertains to skin care and lubricant compositions comprising a compound of the invention.

Still further aspects of the invention are processes which exploit the unique properties of the inventive compounds and articles coated with the inventive compounds.

### Brief Description of the Figures

Figure 1 is a schematic drawing depicting an apparatus for testing lubricant properties of the inventive compounds, as used in Example 3.
Figure 2 is a schematic drawing depicting another apparatus for testing lubricant properties of the inventive compounds, as used in Example 4.
Figure 3 is a graph showing melting point behavior of the product of different ratio mixtures of the components used to produce an exemplary product of the invention, as described in Example 7.

### Detailed Description of the Invention

The compounds of the invention may be produced in several ways. A conventional dialkylquat or trialkylquat salt or hydroxide compound may be mixed with benzoic or salicylic acid or a salt thereof. A water insoluble product is produced which can be used as is. If either starting component is a salt, it is often desirable, depending on the specific application, to wash the product with water to remove counterpart salts or acids which may also be produced by the reaction.

The compounds of the invention have very little or no water solubility over a wide pH range. The effect can be obtained from products prepared over a range of molar ratios of quat cation to aromatic acid anion, generally in the ratio range of from about 0.1:0.9 to 08:0.2, preferably 0.2:0.8 to 0.65:0.35, more preferably from 0.35:0.65 to 0.5:0.5. The optimum silky feel provided by the complexes unexpectedly occurs around the eutectic point of the binary mixture. Maximum effect is near lowest mp euctetic mixture for solids. Some compounds are liquids.

Suitable quat salts which may be used to prepare the compounds of the invention have the formula: 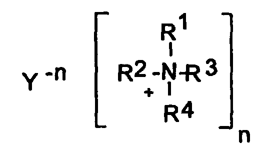 where R¹, R², R³ and R⁴ are as previously defined, Y is a compatible n-valent anion and n is 1, 2 or 3. Examples of suitable groups Y include chloride, bromide, iodide, acetate, methylsulfate, ethylsulfate, sulfate, formate, nitrate, phosphate, tosylate, lactate, citrate, glycolate and mixtures thereof.

Specific quat salts which may be used to prepare the inventive products include dicetyl dimethyl ammonium chloride, dibehenyl dimethyl ammonium chloride, tricetyl methyl ammonium chloride, dilauryl dimethyl ammonium chloride, distearyl dimethyl ammonium chloride, dimyristyl dimethyl ammonium chloride, dipalmityl dimethyl ammonium chloride, and those wherein the C₁₂ to C₃₀ alkyl carbon chain is derived from a tallow fatty acid or from a coconut fatty add. The term "tallow" refers to an alkyl group derived from tallow fatty acids (usually hydrogenated tallow fatty acids), which generally have mixtures of alkyl chains in the C₁₆ to C₁₈ range. The term "coco" refers to an alkyl group derived from a coconut fatty acid, which generally have mixtures of alkyl chains in the C₁₂ to C₁₄ range. Examples or quaternary ammonium salts derived from these tallow and coconut sources include ditallow dimethyl ammonium chloride, ditallow dimethyl ammonium methyl sulfate, di(hydrogenated tallow) dimethyl ammonium chloride, di(hydrogenated tallow) dimethyl ammonium acetate, ditallow dipropyl ammonium phosphate, ditallow dimethyl ammonium nitrate, dicoco dimethyl ammonium chloride, dicoco dimethyl ammonium bromide and mixtures thereof. Examples of salts in which one or more of the R²-R⁴ groups are interrupted with amido and/or ester groups include di(hydrogenated tallowoyl amidoethyl) dimethyl ammonium chloride, methyl bis (tallowoylamidoethyl) 2-hydroxyethyl ammonium methylsulfate, dibehenoyloxyethyl dimethyl ammonium chloride and ditallowoyloxyethyl dimethyl ammonium chloride.

Preferably R² and R³ each have at least 12 carbon atoms. If R² and/or R³ are interrupted with ether oxygen atoms it is preferred that the group contain no more than 5 such atoms, more preferably no more than 2 such atoms. If the ether oxygen forms part of an ethylene oxide moiety it is more preferred that there be no more than one such moiety on the group.

The source of the benzoate or salicylate ion in the inventive compounds may be obtained from benzoic acid or salicylic acid or a salt thereof. Suitable salts are inorganic salts, especially alkaline or alkaline earth salts. The benzoic acid or salicylic acid, or salt thereof, may optionally be substituted with one or more alkyl, alkaryl, halo, alkoxy or hydroxy groups. Preferred compounds of the invention are unsubstituted benzoates or salicylates, most preferably unsubstituted salicylates.

The complexes have very high water repellency and they can greatly reduce the friction of any surfaces in aqueous environments. Their multifunctional properties suggest their feasibility for multifunctional applications. In skin care, they can be used as specialty ingredients, or as a vehicle to carry active ingredients, in barrier creams, water resisting sunscreens and skin smoothing products with silky, non-greasy skin feel. In other industries, they can be used as bioefficacy enhancer for agricultural actives (e.g. glyphosate and dicamba), as lubricants, especially lubricants in aqueous environments, in fabric softeners for fine fabric care, as automotive oil additives, in bathroom mildew preventers, paper debonders and as hydrophobes in car spray formulations.

The skin feel provided by quat-salicylate complexes even surpass the silky skin feel provided by silicones. The soft skin feel is not greasy, yet the inventive compounds imparts a silkiness that renders the compounds useful in a variety of cosmetic applications, as a vehicle and/or as a skin feel enhancing component.

Lubricity imparted to skin or other substrates by the inventive products provides significant additional benefits. In mechanical devices the products may be used as lubricants or as lubricating ingredients in other types of formulations. On skin, abrasion to the hands, feet, genitalia or the like during activities involving rubbing of such body parts is lessened and, in cases of skin-to-skin contact with another person, can improve the sensation for both the wearer of the composition and the other individual. The product consequentially has utility in hand lotions, as a lubricant for condoms and as a vehicle or lubricant component in spermicidal formulations.

In addition to softness and lubricity, the compositions of the invention also impart hydrophobicity to the skin. It does not readily rinse off and appears to be resistant to rinsing with soap. This makes the material especially suitable as a vehicle for UV-absorbers for use in sun-blocks, tanning products and the like. The complex compounds of the invention may also be used as liquid bandages, providing protection to wounds from exposure to water, alone or in mixture with active ingredients such as antiseptics, antibiotics, anti-inflammatories or mixtures thereof.

The combination of lubricity and hydrophobicity also makes the inventive compounds advantageous in swimming and bathing activities, both for reducing drag due to friction at the body/water interface, and for reducing or preventing skin wrinkling caused by extended water exposure. Swimware fabric may also be treated with the inventive complex compounds to reduce friction. Similarly, for mechanical devices which move in water, such as boats, fishing lines and lures, flotation devices, tow ropes, and the like, the inventive compounds can be used as a coating to reduce drag.

As mechanical lubricants the products may be used as is or as additives in automobile oils or other lubricating formulations. Lubricating formulations may be formed free of lithium compounds and/or nitrate compounds with the compounds of the invention.

The compounds of the invention can also be used in car sprays as hydrophobes. In particular, automatic car wash rinse formulations which employ hydrophobe components can be formulated with the inventive compounds as part or all of the hydrophobe component.

Agricultural chemicals formulated with the complex compounds of the invention may produce better adsorption/adhesion on the surfaces of the target substrate, facilitating better uptake of pesticides and herbicides by foliation and insects, less pesticide or herbicide leaching down to the ground, less evaporation of the active ingredient and/or UV protection allowing for longer on-plant life.

It is believed that the products are properly represented as salts since they result from direct mixing of the respective aromatic acid salt and quaternary salt components at ambient temperature. However, the compounds display some properties which are more akin to covalent, rather than ionic compounds. In particular, the compounds, once formed, have a very high hydrophobic property and are surprisingly stable on exposure to a wider range of pH environments than would be expected for a conventional ionic compound. Typically, the salicylate compounds appear to be stable over a pH range of about 2 to about 14, and in some case as low as pH 1. At the lower end of this range, the ionic compound would have been expected to dissociate to give free salicylic acid and at the upper end of the range the quarternary ion would be expected to associate strongly with hydroxide ion and the salicylate ion would be expected to readily extract into an aqueous phase.

If the complex compound of the invention is a solid, it can be used as is, or formulated with other ingredients, in different solid or semi-solid shapes. Thus the compounds can readily be employed in the manner of common stick deodorants and the like. Liquid complexes can be applied directly, e.g. by pouring or from roll-on or spray applicators. Additionally, the complex compounds of the invention, whether solids or liquids, can be formulated into lotions, creams or ointments.

Skin care compositions may be prepared based on the compounds of the invention, optionally in combination with other components. The compositions may be aqueous or non-aqueous.

Aqueous emulsions having a pH of about 5 or higher, preferably 6-10, more preferably about 7-9 may be prepared using suitable surfactants but care must taken in selecting surfactants and their amounts. It has been observed that the beneficial physical properties found in the compounds of the invention, in particular the characteristic skin feel properties can be lost when formulated in compositions which include ethylene glycol or compounds having poly(ethylene oxide) moieties. It is believed that in such compositions, poly(ethylene oxide) surfactants may induce dissociation of the tight complex of the inventive compounds into separate ionic species. Thus, it is preferred that compositions prepared with the complex compounds of the invention be free of compounds with EO functionality. If such compounds are used they should be employed only in certain amounts, typically not more than 10% in formulations and a low EO content is recommended. Emulsifiers with low EO content include ethoxylated alcohols wherein these alcohol derivatives contain a straight or branched chain alkyl group in the C₈₋₃₀, preferably C₁₀₋₂₂, range and which contain from about 1 to about 5 ethylene oxide groups per molecule. Some nonlimiting examples of the low EO emulsifiers include lauryl alcohol ethoxylated with 1 mole of ethylene oxide (i.e. laureth-1), laureth-2, laureth-3, laureth-4, laureth-5, and mixtures thereof. Monoalkylquat salts and amphoteric surfactants are also undesirable as their use can lessen or destroy the unique skin feel and/or the hydrophobic properties provided by the compounds of the invention. If such compounds are used they should be employed only in certain amounts, typically not more than 10% in formulations.

Propylene glycol may be used as a diluent, humectant and/or to facilitate emulsion of the inventive compounds in water. Suitable amounts of propylene glycol range from about 1% to about 50% by weight of the composition. Polypropylene glycols, such as dipropylene glycol, tripropylene glycol and PPGs up to about PPG 50 (*i.e.* polypropylene glycol with an average of about 50 repeat units), and other compounds with poly(propylene oxide) moieties may be used in the skin care compositions in place of or in mixture with propylene glycol.

Skin care products may be aqueous emulsions containing the compositions of the invention. Such emulsions can be formulated with a pH above 4, preferably from about 5 to about 12, more preferably from about 6 to about 10 and most preferably from about 7 to about 9.

Emulsion compositions can span a broad range of consistencies from thin lotions to heavy creams. These emulsions may have viscosities ranging from about 100 cps to about 500,000 cps, preferably from about 3,000 to about 200,000, more preferably from about 5000 cps to about 150,000 cps, and even more preferably from about 5000 cps to about 100,000 cps, as measured at a temperature of 25° C with a Brookfield Viscometer.

Non-aqueous skin care compositions include sunscreen products in which all of the components are oleophilic, and skin coat formulations for swimmers which may be simply consist of one or more of the inventive complex compounds, or may contain additional ingredients which do not interfere with the hydrophobic character of the compounds.

The skin care compositions can comprise a wide variety of optional components in addition to the dialkylquat or trialkylquat benzoate or salicylate component.

In addition to propylene glycol and lower molecular weight polypropylene glycols, other humectants can be used in compositions with the present invention. When used, the humectant can comprise from about 0.1% to about 20%, more preferably from about 0.5% to about 10%, and most preferably from about 1% to about 5% by weight of the composition. Examples of such other humectants useful herein include materials such as urea; guanidine; saturated or unsaturated alkyl α-hydroxy acids and salts thereof, such as glycolic acid and glycolate salts and lactic acid and lactate salts; aloe vera in any of its variety of forms (e.g. aloe vera gel); polyhydroxy alcohols such as sorbitol, glycerol, hexanetriol, butylene glycol, hexylene glycol, and the like; sugar and starch derivatives (e.g., alkoxylated glucose); hyaluronic acid; chitin, starch-grafted sodium polyacrylates; lactamide monoethanolamine; acetamide monoethanolamine; propoxylated glycerol; and mixtures thereof. Even though these materials are defined herein as humectants, they can also possess moisturizing, skin conditioning, and other related properties.

The compositions of the present invention can also include other emollients. Examples of suitable emollients include, but are not limited to, volatile and non-volatile silicone oils (e.g., dimethicone, cyclomethicone, dimethiconol, and the like), highly branched hydrocarbons, and mixtures thereof. The emollients can typically be employed in amounts of from about 0.1% to about 25%, more preferably from about 0.5% to about 10%, and most preferably from about 0.5% to about 5% by weight of the composition.

Other ingredients which can be incorporated into skin care compositions of the present invention in conventional amounts include vitamins and derivatives thereof (e.g. tocopherol, panthenol, and the like); thickening agents; saturated and/or unsaturated alkyl α-hydroxy acids; resins; gums (e.g. guar gum, xanthan gum and the like); waxes (both naturally occurring and synthetic); polymers for aiding the film-forming properties and substantivity of the composition (such as a copolymer of eicosene and vinyl pyrrolidione, an example of which is available from GAF Chemical Corporation as Ganex V-220®); abrasive scrub particles for cleansing and exfoliating the skin; preservatives for maintaining the antimicrobial integrity of the compositions; skin penetration aids such as DMSO, 1-dodecylazacycloheptan-2-one (available as Azone® from Upjohn Co.) and the like; antibiotics, including antimicrobial and antifungal agents; antiinflammatory agents; topical anesthetics; sunscreening additives, such as octyl methoxycinnamate, avobenzone, phenylbenzimidazole sulfonic acid, ZnO and TiO₂; artificial tanning ingredients and tan accelerators such as dihydroxyacetone, tyrosine, tyrosine esters such as ethyl tyrosinate, and phospho-DOPA; skin bleaching (or lightening) agents including but not limited to hydroquinone, kojic acid and sodium metabisulfite; chelators and sequestrants; and aesthetic components such as fragrances, pigments, colorings, essential oils, skin sensates, astringents, skin soothing agents, skin healing agents and the like, nonlimiting examples of these aesthetic components include panthenol and derivatives (e.g. ethyl panthenol), aloe vera, pantothenic acid and its derivatives, clove oil, menthol, camphor, eucalyptus oil, eugenol, menthyl lactate, witch hazel distillate, allantoin, bisabalol, dipotassium glycyrrhizinate and the like.

The invention is illustrated by the following non-limiting examples.

### EXAMPLES

In the following examples the quat-salicylate or quat-benzoate was prepared by mixing the specified ingredients (quat product and acid or its salts) together in the specified proportions, with heating and stirring to melt. An aqueous layer formed if the quat product or the acid or acid salt was dissolved in water. The aqueous layer was separated and discarded if formed. The isolated product, washed if needed, was used as is.

### Skin Feel Test Method

The test method for examples reporting Skin Feel results was as follows:
1) Put - 0.02 gram of sample in a finger and rub it between fingers.
2) Wash the hand with 40°C running water (70 ml/sec) vigorously for> 20 seconds.
3) Feel the lubricity (slipperiness). If it persists> 20 seconds, then it is substantive to skin and has good skin feel.
As a control, a silicone such as Witco organosilicone fluid Silsoft® 148 is used. The slippery feel obtained is considered a good skin feel but without a greasy after feel.

### Example 1

Varisoft® 432 PPG, a solution of 68% dicetyidimethyl ammonium chloride in aqueous propylene glycol sold by Witco Corporation, was reacted with the acids and at the ratios specified in Table 1. Skin feel tests on the products showed a substantial difference between the products of the invention, made from salicylic acid or benzoic acid, and the products made from other acids outside the scope of the present invention.

**Table 1**

| Varisoft® 432 PPG Wt | Acid | Acid Wt | Skin Feel |
|---|---|---|---|
| | | | |

| Invention Examples | | | |
|---|---|---|---|
| 25 g | Salicylic Acid | 6 g | Good |
| 22.14 g | Benzoic Acid | 7.33 g | Good |
| 25 g | Benzoic Acid | 6g | Good |

| Comparative Examples | | | |
|---|---|---|---|
| 4.2 g | Citric Acid (50%) | 3 g | Not Good |
| 4 g | Glycolic Acid (70%) | 1 g | Not Good |
| 25 g | Sebacic Acid | 6 g | Not Good |
| 24.4 g | Palmatic Acid | 10.36 g | Not Good |
| 24.4 g | Palmatic Acid | 5.1 g | Not Good |
| 9.67 g | Stearic Acid | 10.37 g | Not Good |

### Example 2

Salicylic acid was used to prepare quat salicylate salts by mixing with the quat products and at the ratios specified in Table II. Skin feel tests on the salts so obtained showed a substantial difference between the products of the invention, made from a dialkylquat or trialkylquat salt as defined herein, and the products made from quarternary salts outside the scope of the present invention.

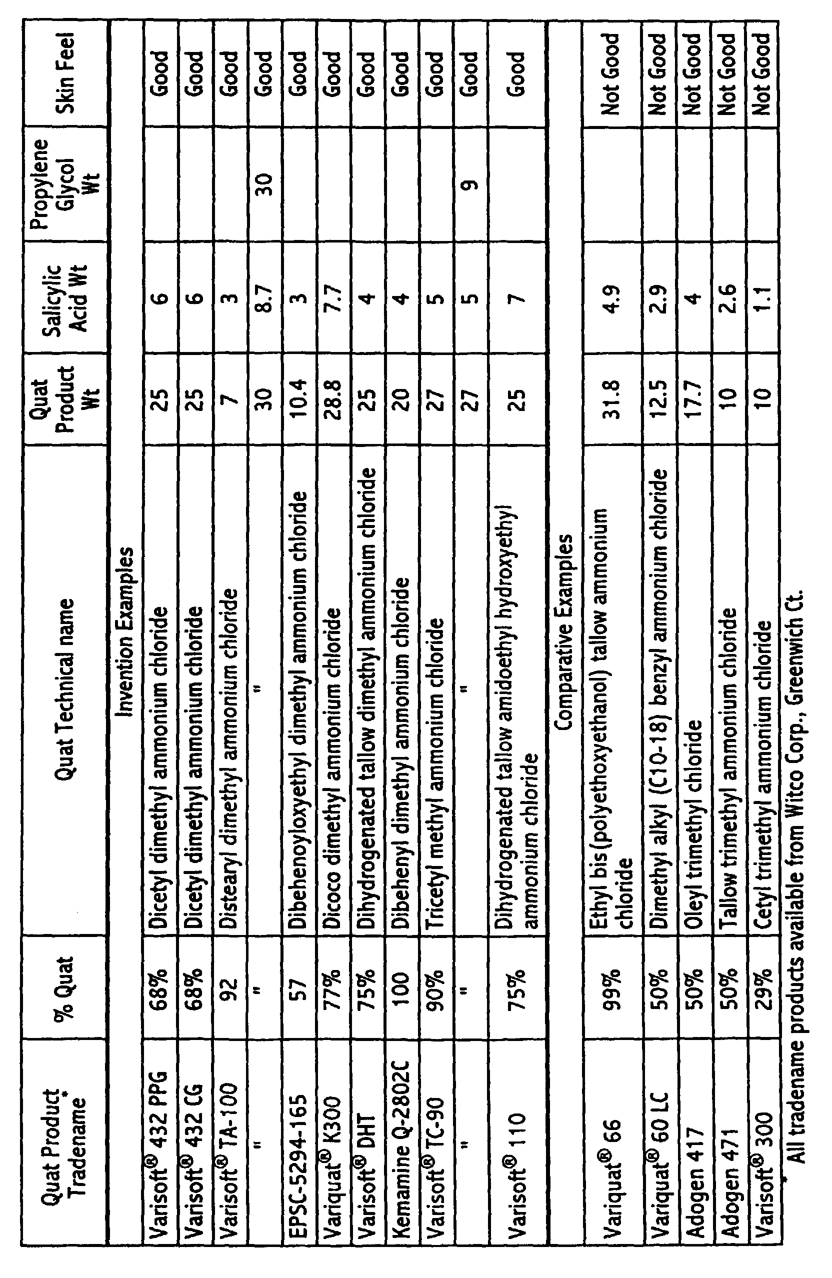

### Example 3

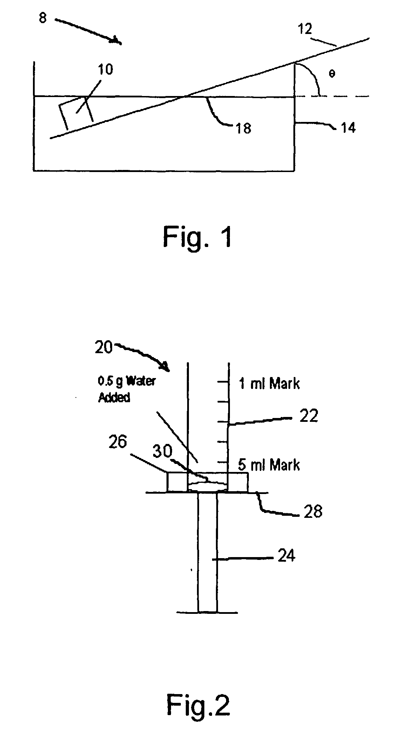

Referring to Fig. 1, there is shown a test apparatus 8 for evaluating surface friction. A rubber wrapped hex nut 10, wrapped with synthetic rubber (material from Evolution One glove), was placed on a glass plate 12. The glass plate 12 was pivoted on the edge of a water tank 14 such that the nut is immersed under the water level 18. The angle θ was increased until the hex nut started to fall. The falling angle was 19°.

The experiment was repeated except that a quat-anion complex compound of the invention prepared from 25 g Varisoft® 432 CG and 6 g salicylic acid was used to treat the surfaces before immersion. To treat the surfaces the quat-anion complex was applied to the rubber surface, and rubbed against the glass surface, then hex nut and glass plate were rinsed with warm water to get rid of excess quat-salicylate. The falling angle was reduced to 11°, demonstrating that the inventive complex reduced the friction between the glass and rubber surfaces.

### Example 4

Referring to Fig. 2 there is shown a test apparatus 20 for evaluating surface friction. The apparatus was prepared by cutting the tip off of a 5 ml syringe (Slip Tip Syringe by Becton & Co.), disassembling the syringe, cleaning the components with soap and rinsing. The syringe was reassembled and mounted such that the barrel 22 was above the plunger 24. The plunger 24 was pulled back fully so that the barrel was empty. 0.5 g of water was placed in the barrel and a weight 26 (several washers) was mounted on the barrel flange 28. This is the initial configuration as shown in Figure 2. The weight was selected to be sufficient to cause the syringe barrel to slide down the plunger. Using 312 grams of weight the time for the plunger tip 30 to reach the 1 ml mark was 27 seconds.

The syringe was disassembled and the same quat complex compound of the invention used in Example 3 was applied to the plunger tip 30 and to the inner surface of the barrel 22. The surfaces were rinsed with warm water to get rid of excess quat-salicylate before the syringe was reassembled in the manner described above. Using only 225 grams of weight the time for the plunger tip 30 to reach the 1 ml mark was only 10 seconds.

### Example 5

Melting the mixture of 3 grams of salicylic acid and 7 g of Varisoft® TA-100 resulted in a solid at room temperature. This quat-salicylate complex is very substantive to skin. It gave very good emolliency and silky/slippery feel to skin when applied the complex together with some propylene glycol to skin. The starting components, Varisoft® TA-100 and salicylic acid alone did not show same emolliency or skin feel. When 10-20% by weight of this complex was stirred in water, the complex dispersed without gumming up, indicating that the material can be formulated successfully into lotions and creams. Dispersions in water at several pH values in the range of 1-14, when applied to the skin and rinsed, gave the same skin feel. The silky/slippery skin feels also existed when the quat-salicylate complex was applied to skin in mixture with isopropyl alcohol, ethyl alcohol, glycerin, homosalate, or mineral oil.

### Example 6

The mixture product obtained from adding 6 grams of salicylic acid into 25 g of Varisoft® 432 PPG was a clear low viscosity liquid at room temperature with light yellow/brown color. Its behavior in water, on the skin and in mixture with IPA, ethyl alcohol, glycerine, homosalate, or mineral oil was similar to the complex product produced in Example 5.

### Example 7

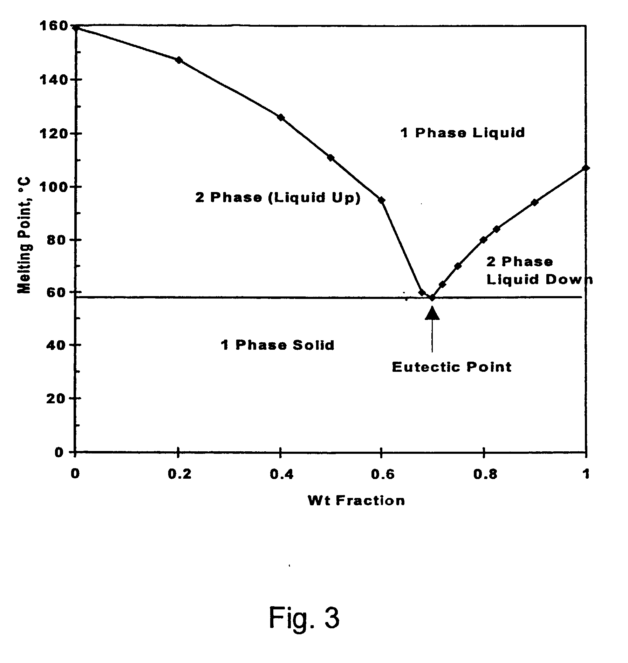

Reaction products of salicylic acid and Varisoft® TA-100 were prepared at different weight fractions of Varisoft® TA-100/(salicylic acid + Varisoft® TA-100). Figure 3 depicts the melting point curve of the mixture products tested. The mixtures which produced the optimum silky/slippery skin feel were observed to occur around the eutectic point (minimum melting point) of the binary mixture. Further it was observed that the binary mixture at the eutectic composition has a much lower melting point: 57°C lower than that of TA-100 and 100°C lower than that of salicylic acid. These results, together with the independence of skin feel and water repellency from pH as demonstrated in Example 5, are considered to indicate that an unusually strong interaction between the quaternary ammonium cation and the salicylate anion is responsible for the unique silky skin feel of the inventive products.

### Example 8

Two stock solutions were prepared as follows:
- Solution A: 1.5 g sodium salicylate was added into 98.5 g deionized water at room temperature with stirring.
- Solution B: 7.29 g Varisoft® 432 CG was added into 142.7 g deionized water at room temperature with stirring.
The above solutions had the same molar equivalents.

Solution B was poured slowly into Solution A with stirring at room temperature. A white precipitate was obtained. The white precipitate was a gummy substance with very low solubility in water and very slippery feel. The precipitate then was washed with 100 g DI water to wash away residual sodium chloride. The precipitate was mixed with 100 g water and heated with stirring up to 75 -80 °C. A two phase system was obtained with the top phase being a gummy clear liquid with a light brownish color. The mixture was cooled to room temperature and the bottom water phase removed. The aqueous insoluble product weighed about 7 g.

The product was placed in a 105°C oven for 2 hours to dry off water. About 5.6 gram of dry product (a clear viscous liquid with light brownish color) was obtained, which is dicetyldimethyl ammonium salicylate. The dicetyldimethyl ammonium salicylate has very slippery hand feel and very low solubility in water. These properties were retained in the presence of water having a pH as low as 1.

The above examples and disclosure are intended to be illustrative and not exhaustive. These examples and description will suggest many variations and alternatives to one of ordinary skill in this art. All these alternatives and variations are intended to be included within the scope of the attached claims. Those familiar with the art may recognize other equivalents to the specific embodiments described herein which equivalents are also intended to be encompassed by the claims attached hereto.

## Claims

1. A compound of the formula: 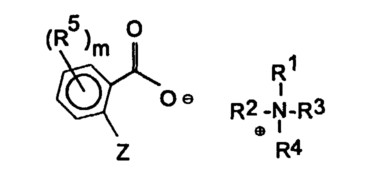 where R¹ is an alkyl group having 1 to 4 carbon atoms, which may optionally contain a hydroxyl group, R² and R³ are independently alkyl or alkenyl groups, which may be optionally interrupted with ester, ether oxygen and/or amido linkages, the R² and R³ groups having 8-30 carbon atoms, R⁴ is a group as defined for R¹ or for R², Z is H or OH, R⁵ is alkyl, alkaryl, aralkyl, halo, alkoxy, hydroxy and m is 0-4.

2. A compound as in claim 1 where Z is OH.

3. A compound as in claim 1 where R⁵ is H.

4. A compound as in claim 1 wherein R¹ is methyl and R² and R³ are selected from the group consisting of cetyl, lauryl, behenyl, stearyl, myristyl, palmityl, tallow, hydrogenated tallow, hydrogenated tallow amidoethyl, behenoyloxyethyl, tallowyloxyethyl and coco.

5. A compound as in claim 4 wherein R⁵ is H.

6. A compound as in claim 5 where Z is OH.

7. A chemical composition consisting essentially of a compound as in claim 1, or a mixture thereof.

8. A skin care composition as in claim 1 comprising a compound as in claim 1.

9. A skin care composition as in claim 8 further comprising at least one of a humectant, a vitamin, a thickening agent, an α-hydroxy acid, a resin, a gum, a film former, abrasive scrub particles, an antibiotic, an antiinflammatory agent, an anesthetic, a tanning agent, a skin bleaching agent, a sunscreen, a chelator or a sequestrant.

10. A skin care composition as in claim 8 further comprising at least one of a fragrance, a pigment, a dye, a preservative, an essential oil, a skin sensate, an astringent, a skin smoothing agent or a skin healing agent.

11. A skin care composition as in claim 8 being substantially free of water.

12. A skin care composition as in claim 11 further comprising a sunscreen agent.

13. A skin care composition as in claim 8 wherein said compound is dispersed in water having a pH of about 5 or greater.

14. A skin care composition as in claim 13 wherein the aqueous dispersion pH is in the range of 6-12.

15. A skin care composition as in claim 14 wherein the aqueous dispersion pH is in the range of 7-9.

16. A hard surface disinfectant composition comprising a disinfectant agent in a vehicle, wherein the vehicle comprises a compound as in claim 1.

17. A hard surface disinfectant as in claim 16 wherein the vehicle consists essentially of said compound.

18. A process for disinfecting a hard surface comprising wiping thereon a composition as in claim 16.

19. A composition comprising a compound as in claim 1 and an herbicide or pesticide.

20. A process of applying a biological active to an object comprising applying to said substrate a composition of said active in a carrier, wherein the carrier comprises a compound as in claim 1.

21. A process as in claim 21 wherein said biological active is an antibacterial or antifungal agent.

22. A process as in claim 20 wherein said object is a plant and said biological active is an herbicide or a pesticide.

23. A process comprising a step in which an object is moved through a body of water, wherein the object has, on at least a portion of the water contacting surfaces thereof, a coating comprising a compound as in claim 1.

24. A process as in claim 23 wherein the object is a swimmer and the coating is applied to exposed skin surfaces thereof.

25. A process as in claim 23 wherein the object is a member of the group consisting of boats, fishing line, fishing lures, flotation devices, and ropes.

26. A process for lubricating moving surfaces in a mechanical device, process comprising applying a composition comprising a compound as in claim 1 between said moving surfaces.

27. A process as in claim 26 wherein the composition is free of lithium and nitrate compounds.

28. A method of providing a skin-care composition having a pH of greater than 5 with a non-greasy silky skin feel, the method comprising adding to the composition a compound as in claim 1.

29. A method for providing enhanced water repellancy comprising identifying a surface on which an enhanced water repellancy characteristic is desired and applying a composition comprising a compound as in claim 1 to the surface.

30. A compound as in claim 1 where R² and R³ each have at least 12 carbon atoms.

31. A water insoluble composition obtained from a mixture consisting essentially of
a.) one or more compounds of the formula: 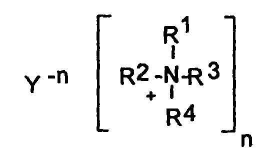 where R¹ is an alkyl group having 1 to 4 carbon atoms, which may optionally contain a hydroxyl group, R² and R³ are independently alkyl or alkenyl groups, which may be optionally interrupted with ester, ether oxygen and/or amido linkages, the R² and R³ groups having 8-30 carbon atoms, R⁴ is a group as defined for R¹ or for R², Y is chloride, bromide, iodide, acetate, methylsulfate, ethylsulfate, sulfate, formate, nitrate, phosphate, tosylate, lactate, citrate, glycolate or a mixture thereof and n is 1, 2 or 3; and
b.) one or more of benzoic acid or a salt thereof, or salicylic acid or a salt thereof.

32. A composition as in claim 31 wherein the component a.) is a member of the group consisting of dicetyl dimethyl ammonium chloride, dibehenyl dimethyl ammonium chloride, tricetyl methyl ammonium chloride, dilauryl dimethyl ammonium chloride, distearyl dimethyl ammonium chloride, dimyristyl dimethyl ammonium chloride, dipalmityl dimethyl ammonium chloride, and ditallow dimethyl ammonium chloride, ditallow dimethyl ammonium methyl sulfate, di(hydrogenated tallow) dimethyl ammonium chloride, di(hydrogenated tallow) dimethyl ammonium acetate, ditallow dipropyl ammonium phosphate, ditallow dimethyl ammonium nitrate, dicoco dimethyl ammonium chloride, dicoco dimethyl ammonium bromide, di(hydrogenated tallowoyl amidoethyl) dimethyl ammonium chloride, ditallowoyloxyethyl dimethyl ammonium chloride, dibehenoyloxyethyl dimethyl ammonium chloride and mixtures thereof.

33. A composition as in claim 32 wherein the component b.) is an inorganic salt of benzoic or salicylic acid.

34. A composition as in claim 31 wherein, after mixing the components a.) and b.), the resulting water insoluble product has been separated from water and water soluble components.

35. A composition as in claim 34 wherein said mixing is accomplished in aqueous dispersion at a pH above 4.

36. A rinse formulation for an automatic car wash comprising a dispersion of a hydrophobe in water, the hydrophobe comprising a compound as in claim 1.

37. A chemical composition which includes a compound as in claim 1 and which is substantially free of water.
